# EUROPÄISCHE PATENTANMELDUNG

(11) **EP 1 121 951 A1**
(43) Veröffentlichungstag der Anmeldung: **08.08.2001**
(21) Anmeldenummer: 01101892.6
(22) Anmeldetag: 27.01.2001
(51) Int. Cl.: A61M 16/00, A61M 1/00, A61B 5/00, G05B 19/00, A61M 3/00, A61M 5/00, A61M 16/20

(54) **Regelkreis - System zur Therapie eines Patienten**

(30) Priorität: 01.02.2000 DE 10004230; 03.02.2000 DE 10004653; 05.05.2000 DE 10021843; 21.09.2000 DE 10046740
(71) Anmelder: Hölscher, Uvo M., Prof. Ph. D., 48565 Steinfurt (DE)
(72) Erfinder: Hölscher, Uvo M., Prof. Ph. D., 48565 Steinfurt (DE)
(74) Vertreter: Habbel, Ludwig (Lutz), Dipl.-Ing.

(57) **Zusammenfassung**

Die Erfindung bezieht sich auf ein Regelkreis - System zur Therapie eines Patienten, mit einer Dosiereinheit, und mit einer Meßvorrichtung und mit einer Stellvorrichtung, wobei die Dosiervorrichtung als Einzelsystem sicher konstruiert ist, wobei an der Dosiervorrichtung eine maximale Therapiegröße einstellbar ist, und die Stellvorrichtung die Therapiegröße in Richtung des sicheren Zustandes verändern kann, aber auf Werte beschränkt ist, die von der maximalen Therapiegröße und dem sicheren Zustand begrenzt sind.

## Beschreibung

### Problemfeld:

Systeme in der Medizintechnik, die zB aus mehreren Komponenten bestehen können, dürfen auch im Fall eines ersten Fehlers keine Gefahr für den Patienten oder den Anwender bedeuten.

Geregelte Systeme, die zB einen physiologischen Parameter des Patienten beeinflussen sollen, zB durch die Dosierung eines Medikaments, müssen aus diesem Grund folgende Besonderheit aufweisen:
◆ Sie müssen gegenüber dem unbemerkten Auftreten eines ersten Fehlers sicher aufgebaut sein
◆ Es muß für ein derartiges System der sichere Zustand für den Fall eines Versagens definiert sein.

Dies hat zur Folge, dass i.a. die Subsysteme des gesamten Regelkreises entweder zweikanalig aufgebaut sein müssen (ein zweiter Kanal kontrolliert den ersten auf Intaktheit) oder durch aufwendige weitere Überwachungsvorrichtungen muss permanent auf ordnungsgemässes Funktionieren überprüft werden. Dies kann zB dadurch geschehen, dass für den zu stabilisierenden Parameter ein Monitor eingesetzt wird, der diesen Parameter dem Regler als Regelgröße/lstgröße zur Verfügung stellt, daneben aber ein unabhängiger, zweiter Monitor zur Überwachung eingesetzt wird, der das Funktionieren des ersten Monitors und des Reglers überwacht. Darüber hinaus ist meist eine weitere Sicherheitsschaltung notwendig, die automatisch bei Diskrepanz der Werte von den beiden Monitoren Alarm gibt und das System in den sicheren Zustand überführt. Damit bekommt das Gesamtsystem eine hohe Komplexität. Dies impliziert einen sehr hohen technischen Aufwand sowie grosse Kosten. Deshalb wird bislang kein solches System kommerziell angeboten, da es in einer sicherheitstechnisch einwandfreien Konzeption wirtschaftlich nicht zu fertigen ist. Z.Z. kommen lediglich Systeme mit erheblichen Sicherheitsdefiziten für Forschungszwecke (zB in der Anästhesie) zum Einsatz, die der ständigen Kontrolle eines erfahrenen Arztes bedürfen und deshalb weder nach den Regularien der Medical Device Directive / Europa oder den Vorschriften der FDA / USA zugelassen werden können.

### Anästhesie:

Patienten im OP werden hypnotisiert, um das Bewußtsein und die Wahrnehmung bei der Narkose auszuschalten.

In der Narkose ist es üblich, daß man den hypnotischen Anteil der Medikamentierung nach der gewünschten Anästhesietiefe steuert. Den während der Narkose rasch wechselnden Anforderungen an die Hypnosetiefe wird entweder durch variable Dosierung durch den Anästhesisten entsprochen. Ziel ist es, Medikamente einzusetzen, die eine schnelle Anpassung der Hypnosetiefe an den erforderlichen Level ermöglichen (schnelle Anflutung) sowie ein schnelles Aufwachen ermöglichen. Diese Medikamente haben die Eigenschaft, dass ihre Dosierung sich meist nicht zeitlich konstant durchführen lässt sondern in Abhängigkeit von der Umverteilung im Körper in Regionen, die verschieden schnell und viel vom Medikament aufnehmen, angepasst werden muss (Pharmakokinetik).

Es stehen die intravenöse und inhalative Anästhetika zur Wahl.

### IV-Anästhesie:

Bei der intravenösen Anästhesie wird in aller Regel das Medikament über eine Infusionspumpe kontinuierlich dem Patienten intravenös verabreicht. Eine repititive Gabe von Boli (grösseren Mengen in einem einzigen Schritt mit Pausen zwischen den Dosierungen) empfiehlt sich nicht, weil dadurch wechselnde Medikamentenspiegel im Blut und damit wechselnde Hypnosetiefen einhergehen.

Infusionspumpen sind sicherheitstechnisch so ausgelegt, dass die Förderung mit der eingestellten Rate der Normalzustand ist. Fällt die Pumpe aus oder tritt ein Fehler auf, muss die Infusion gestoppt werden. Der Anästhesist kann die Narkose manuell weiterführen. Dieser Zustand wird auch als der sichere Zustand definiert.

### BET Schema:

Um schnell auf einen adäquaten Blutspiegel des Medikamentes zu kommen, wurde früher die Infusatmenge nach dem BET Schema bemessen. Ein initialer Bolus dient dazu, im Blut schnell eine entsprechenden Wirkkonzentration zu erreichen. Danach wird eine zeitlich abfallende Menge appliziert, die bis auf einen Wert zurückgeht, der ausreicht, um die Narkose aufrecht zu erhalten. Dieser Wert wird erst nach langer Zeit erreicht, wenn Umverteilungsvorgänge des Medikamentes im Körper kaum noch eine Rolle spielen. Muss die Narkose vertieft werden, muss ein ähnlicher Prozess erneut angeschlossen werden. Da die exakte Berechnung der erforderlichen Mengen durch Kopfrechnen nicht möglich ist, die resultierende Funktion ist eine Überlagerung verschiedener e-Funktionen, wurde üblicherweise in einem praktisch erprobten Stufenprofil dosiert.

Zusammenfassend kann gesagt werden, dass eine Dosierung, die kurzfristig einen Konzentrationslevel im Körper erreichen und nachfolgend aufrechterhalten will, über gewisse Zeitabschnitte ein vielfaches der Rate geben muss, die über lange Zeit gegeben als unbedenklich eingestuft wird. Die kurzfristige, grosse Überschreitung des sicheren Bereiches ist nur erlaubt, um die dynamischen Vorgänge (Auffüllen verschiedener Speicher im Körper) zu kompensieren.

Solche Schemata finden immer dann Anwendung, wenn ein Medikament auf konstantem Wirklevel gehalten werden soll. Dies ist neben der Anästhesie auch in anderen Anwendungsgebieten der Fall.

### TCl:

Die Verfügbarkeit mikroprozessorgesteuerter Infusionspumpen erlaubt heute die laufende Berechnung und Anpassung der erforderlichen Infusionsmenge. Eingestellt wird an einer solchen Pumpe ein gewünschter Konzentrationslevel des Anästhetikums im Körper des Patienten (Blutlevel oder Level im Effekt-Kompartment). Da die Eigenschaften der Medikamente und der Patienten unterschiedlich sind, verarbeitet die TCI-Pumpe üblicherweise die Patienten-Informationen: Geschlecht, Alter, Gewicht, Temperatur, ... sowie die Medikamentenspezifika, um die individuelle Infusionsrate zu berechnen.

Pumpen, die diese Funktion enthalten, werden mit dem Kürzel TCI (Target Controlled Infusion) bezeichnet. Die TCI-Pumpe erhöht also zu Beginn einer Infusion und nach jeder Vertiefung des Levels selbständig für kurze Zeit die Infusionsrate auf Werte deutlich oberhalb der Rate, die über lange Zeit gegeben als unbedenklich eingestuft wird. Sicherheitstechnisch wird dies so gelöst, dass die Ratenberechnung, die die kurzfristige "Übersteuerung" bewirkt, in der Pumpe zweikanalig durchgeführt wird. Die TCI-Pumpe ist damit sicherheitstechnisch Stand der Technik.

### Regelkreise:

Die individuell erforderliche Konzentration von Anästhetikum im Blut ist unterschiedlich. Konventionell werden klinische Zeichen wie Blutdruck, Herzfrequenz, Hautfeuchte ... zur Ermittlung des anästhetischen Zustandes herangezogen. Neuere Konzepte setzen physiologische Parameter ein, um die Hypnosetiefe zu bestimmen. Der Messwert kann dann in einem Regelkreis herangezogen werden, die notwendige Dosierung zu erzielen.

Als Eingangsparameter für die Regelung kann z.B. die Gehirnstromaktivität des Patienten (EEG) dienen (Schüttler, Schwilden et al.) oder evozierte Potentiale (Kenny).

Es ist Ziel des vorliegenden Anmeldungsgegenstandes, ein Konzept für die sicherheitstechnische Ausgelegung von Systemen zu beschreiben, in denen die notwendige Zweikanaligkeit bzw. das Sicherheitskonzept sich nur auf eine Untereinheit des Gesamtsystemes erstrecken braucht, ohne dass die Sicherheit des Gesamtsystems darunter leidet.

### Ratenmodulation:

Bezogen auf die automatisierte Langzeitsedierung (Überwachung der Sedierungstiefe zB mittels einer einkanaligen Monitoreinrichtung, zB EEG) kann mit einer zweikanligen konventionellen Therapieeiheit, also einer in sich sicheren Einheit (zB Infusionspumpe) das Sedativum verabreicht werden. Hierbei wird die Infusionsrate an der Pumpe auf einen unbedenklichen Wert eingestellt, der der üblichen ärztlichen Dosierung entspricht. Der Monitor kann mit Hilfe des Reglers lediglich die Infusionsrate, zB durch Pulsbreitenmodulation, auf Werte kleiner der eingestellten Rate regeln, zB indem die Pumpe kurzfristig angehalten wird. Da der Stillstand der Pumpe als sicherer Zustand angesehen wird und die volle eingestellte Infusionsrate auch (ärztlich gewollte Rate) kann das Gesamtsystem sich nur zwischen den beiden Zuständen bewegen und ist damit ingesamt sicher.

### Modulation einer Zwischengrösse:

Das System soll aber möglichst aber auch eine zeitlich begrenzte Dosierung deutlich oberhalb der Rate ermöglichen, die über lange Zeit gegeben als unbedenklich eingestuft wird, um dynamische Vorgänge besser ausregeln zu können.

Eingesetzt werden zB eine konventionelle TCI-Infusionspumpe, ein Regler und ein oder mehrere physiologische Monitore.

Die TCI-Pumpe besitzt die bekannte Einstellmöglichkeiten, mit der ein Soll-(Blut)Level - im nachherein als maximaler Level bezeichnet - eingestellt werden kann. Der maximale Wirklevel an der TCI-Infusionspumpe wird durch den Anwender auf den im klassischen Betrieb üblichen Wert eingestellt, der im ärztlichen Ermessen den maximalen Blutlevel für die Narkose darstellt.

Erfindungsgemäss besitzt die TCI-Pumpe zusätzlich eine Eingangsschaltung, die es erlaubt, den eingestellten Blutlevel abzusenken. Damit wird zB beim Ausbleiben des Signals am Eingang der Blutlevel auf Null abgesenkt. Liegt das volle Signal an, wird lediglich der an der TCI-Pumpe eingestellte maximale Level dosiert.

Die Eingangsschaltung kann zB über eine Pulsbreitenmodulation oder über andere Techniken funktionieren. Wichtig ist nur, dass der effektive Level, den die TCI-Pumpe als Steuergrösse hat, lediglich zwischen dem sicheren Zustand (zB Null) und dem eingestellten, maximalen Level schwanken kann.

Der Regler wertet die Signale in einer solchen Weise aus, daß er bei Absinken des Indikators für zB die Schlaftiefe (Patient wird wacher) unter den eingestellten Regel-Sollwert das Eingangssignal für die Infusionspumpe erhöht. Hat der Indikator für die Schlaftiefe wieder einen Sollwert erreicht, hält der Regler den Level wieder konstant.

Der Regler hat somit das typische Verhalten, dass die effektive Stellgröße, hier der Blutlevel, nur Werte zwischen zwei definierten Zuständen annehmen kann, die auch unter Annahme eines ersten Fehlers keine Gefährdungen für den Patienten darstellen.

Vorteilhaft wird durch diesen Regelkreis bei geringem Schmerzreiz oder bei individuell hoher Wirkung von geringen Medikamentenmengen die Medikamentenzufuhr auf das erforderliche Minimum verringert.

Das Sicherheitskonzept wird in dem Regelkreis dahingehend ausgenutzt, daß der Arzt den maximal sinnvollen Level vorgibt, der nicht schädigen kann, und unter normalen Aspekten den gewünschten Effekt (zB die Hypnosetiefe) garantiert.

Der Regler selber kann den Level lediglich in Richtung des sicheren Zustandes verändern (hier verringern). In der worst case Betrachtung ist im Fehlerfall damit lediglich der sichere Zustand oder der maximale ärztlich tolerable Level (hier die Unterdosierung oder die Maximaldosierung, nie aber eine Überdosierung) möglich. Da sowohl die vom Arzt vorgegebene Maximaldosierung als nicht gefährdend angesehen werden kann als die Nullförderung als sicherer Zustand betrachtet wird, besitzt der gesamte Regelkreis trotz fehlender Redundanz des Monitoring die Sicherheitsstufe eines zweikanaligen Konzeptes.

Zusätzlich kann eine zeitliche Bewertung der Stillstandsphase der Infusionspumpe entsprechende Abweichungen vom erwartungsgemäßen Verhalten des Infusionsverlaufs aufdecken und durch eine entsprechende Alarmgabe auf die vorliegende oder sich anbahnende Unterdosierung hinweisen.

Erfindungsgemäß stellt die Erfassung des Dosierverlaufes und das daraus resultierende Infusionsprofil eine zusätzliche therapeutische Information dar, die der Therapie aus dokumentarischen Gründen zur Verfügung gestellt werden können.

Damit ergibt sich ein extrem kostengünstiger Aufbau, der einen zweiten physiologischen Monitor überflüssig macht. Außerdem kann der Regler einkanalig aufgebaut werden.

Vorteilhaft muß die Meßvorrichtung und/oder die Stellvorrichtung nicht aus sicherheitstechnischen Gründen auf korrektes Funktionieren geprüft werden.

Der Regelkreis kann für nahezu beliebige Wirkzusammenhänge wie Blutdruck, Relaxation o. dgl. Anwendung finden und mit entsprechenden Monitoren zusammenwirken.

Der Regelkreis kann für nahezu beliebige Therapiegeräte wie Infusionsgeräte, Reizstromgeräte, Narkosemittelverdampfer, Gasdosierer, o. dgl. Anwendung finden.

Als "sicherer Zustand" ist nicht notwendigerweise ein für den Patienten optimaler Zustand bezeichnet, mit der entsprechenden optimalen Dosis, sondern es kann in den wahrscheinlich meisten Fällen beispielsweise eine "Dosierung = Null" vorgesehen sein wie z. B. bei Pumpen; je nach Therapiegerät kann jedoch auch eine Dosierung vorgesehen sein, die über dem Maximum liegt, welches während des Regelbetriebes eingestellt werden kann.

Die grundsätzliche Idee, daß an der Dosiervorrichtung eine maximale Therapiegröße einstellbar ist, und daß die Stellvorrichtung die Therapiegröße in Richtung des sicheren Zustandes verändern kann, aber auf Werte beschränkt ist, die von der maximalen Therapiegröße und dem sicheren Zustand begrenzt sind, kann dahingehend ausgeführt werden, daß beispielsweise die von der Dosiereinheit abzugebende Therapiegröße überwacht wird, oder daß beispielsweise die beim Patienten gemessenen Werte wie z. B. Atmung, Pulsfrequenz oder Blutdruck überwacht werden und zugunsten einer Veränderung der Dosierung ausgewertet werden.

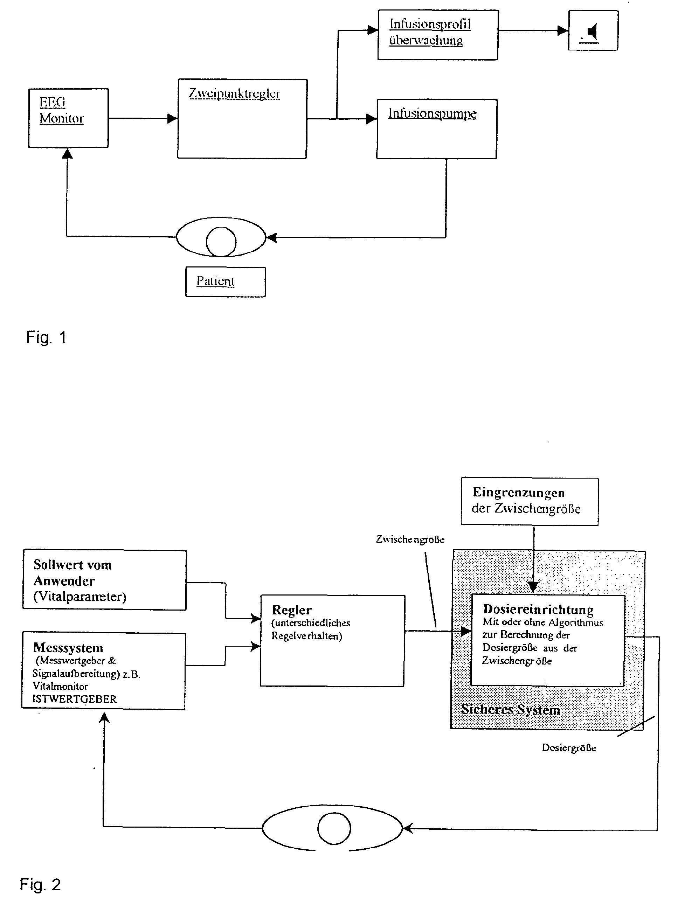

Zwei Ausführungsbeispiele der Erfindung sind in den Fig. 1 und 2 dargestellt.

## Patentansprüche

1. Regelkreis - System zur Therapie eines Patienten,
mit einer Dosiereinheit,
die als Eingangsgrösse einen Wert benötigt, der zur Steuerung der Therapie in die Dosiergröße umgerechnet wird und dabei technische Eigenschaften des Systemes oder physiologische Eigenschaften des Patienten oder Eigenschaften des Therapeutikums wie pharmakologische, physikalische oder chemische Eigenschaften berücksichtigt,
und mit einer Meßvorrichtung
zur Aufnahme von Signalen und Meßwerten vom
Patienten oder vom System
und mit einer Stellvorrichtung,
die die Signale von der Meßvorrichtung so auswertet, daß die Dosierung durch die Dosiereinheit zu einem gewünschten Zustand des Patienten oder im System führt,
wobei die Dosiervorrichtung als Einzelsystem sicher konstruiert ist,
dadurch gekennzeichnet,
daß an der Dosiervorrichtung eine maximale Therapiegröße einstellbar ist,
und daß die Stellvorrichtung die Therapiegröße in Richtung des sicheren Zustandes verändern kann, aber auf Werte beschränkt ist, die von der maximalen Therapiegröße und dem sicheren Zustand begrenzt sind.

2. System nach Anspruch 1,
dadurch gekennzeichnet,
daß die Dosiereinheit zur Verabreichung von Medikamenten ausgestaltet ist, wie in das Blut eines Menschen, und daß die maximale Dosiergröße der Dosiervorrichtung einstellbar ist,
und daß die Stellvorrichtung die Dosiergröße in Richtung des sicheren Zustandes verändern kann, aber auf Werte beschränkt ist, die von der maximalen Dosiergröße und dem sicheren Zustand begrenzt sind.

3. System nach Anspruch 1 oder 2,
dadurch gekennzeichnet,
daß die Dosiereinheit (1) als TCI-Dosiereinheit (1) zur Verabreichung von Medikamenten ausgestaltet ist, wie in das Blut des Menschen,
wobei die notwendige Dosierung zur Erzielung eines konstanten Wirkstofflevel im Patienten durch pharmakokinetische oder pharmakodynamische Berechnung vorgegeben ist,
und daß dieser maximale Wirkstofflevel einstellbar ist,
und daß die Stellvorrichtung den Wirkstofflevel in Richtung des sicheren Zustandes verändern kann, aber auf Werte beschränkt ist, die von der maximalen Dosiergröße und dem sicheren Zustand begrenzt sind.

4. System nach einem der vorhergehenden Ansprüche, da
durch gekennzeichnet, daß
die Stellvorrichtung und / oder die Messeinrichtung sicherheitstechnisch einkanalig ausgeführt ist.

5. System nach einem der vorhergehenden Ansprüche, gekennzeichnet durch eine Überwachungsschaltung, welche durch eine zeitliche Bewertung des Therapieprofils sich anbahnende Fehldosierungen berechnet und dies mittels einer Anzeigevorrichtung frühzeitig signalisiert.

6. System nach einem der vorhergehenden Ansprüche, gekennzeichnet durch eine Auswertungsschaltung, welche aus dem zeitlich gemittelten Therapieprofil zusätzliche ärztliche Informationen zu den Therapieiephasen und zum Patienten berechnet und mittels einer Anzeigevorrichtung ausgibt.

7. System nach Anspruch 5, gekennzeichnet durch eine Überwachungsschaltung und Anzeige, zur Ableitung eines Indikators für die individuelle Empfindlichkeit des Patienten in Bezug auf das dosierte Medikament.

8. System nach Anspruch 5, gekennzeichnet durch eine Überwachungsschaltung und Anzeige, zur Ableitung von therapeutischen Informationen zu Wach- & Schlafphasen aus einem Infusionsprofil.

9. System nach Anspruch 5, gekennzeichnet durch eine Überwachungsschaltung und Anzeige, zur Ableitung von medizinischen Informationen zu therapeutischen Zielen aus dem Therapieprofil.

10. System nach einem der vorhergehenden Ansprüche, gekennzeichnet durch
eine Prüfschaltung zur Prüfung der Integrität der Stellvorrichtung durch die Dosiereinheit derart,
dass die Stellvorrichtung in regelmässigen Abständen ein codiertes Signal liefern muss,
andernfalls die Pumpe alarmiert und z.B. einen vorgegebenen Wert ansteuert oder den derzeitigen Wert beibehält.

11. System nach einem der vorhergehenden Ansprüche, gekennzeichnet durch
eine Auswertungsschaltung zur Prüfung der Integrität der Stellvorrichtung durch die Dosiereinheit derart,
dass die Stellvorrichtung nur den Level zwischen dem eingestellten maximalen Level und einem unteren Grenzwert verstellen kann,
wobei sich Ausgangssignal zwischen 100% und
einem unteren Grenzwert ändert,
andernfalls die Dosiereinheit den Anwender alarmiert.

12. System nach einem der vorhergehenden Ansprüche, dadurch gekennzeichnet, daß
die Dosiereinheit einen Speicher für Charakteristika des zu verwendenden Therapeutikums aufweist,
sowie Eingabemittel zum Einlesen von derartigen Charakteristika in den Speicher,
sowie eine Auswertungsschaltung, welche in Abhängigkeit der abgespeicherten Charakteristika Grenzwerte für die Therapie vorschlägt oder fest vorgibt.

13. System nach einem der vorhergehenden Ansprüche, gekennzeichnet durch
eine Prüfschaltung zur Prüfung der Integrität des Regelkreises durch die Stellvorrichtung derart,
dass der Stellvorrichtung Wertetabellen mit typischen Werten für die Empfindlichkeit des Patienten, des Systems oder des Therapeutikums zugeordnet sind,
in Form einer Abhängigkeit des physiologischen
Parameters von dem Dosislevel,
und daß die Prüfschaltung die aktuellen Werte mit diesen Wertetabellen vergleicht.

14. System nach einem der vorhergehenden Ansprüche, gekennzeichnet durch
eine Sicherheitsschaltung,
welche der Stellvorrichtung zugeordnet ist und dem akuellen Patienten oder System ein Empfindlichkeitsprofil zuordnet,
und welche mit Hilfe dieses Empfindlichkeitsprofils den späteren Regelverlauf ständig bewertet.

15. System nach einem der vorhergehenden Ansprüche, gekennzeichnet durch
eine Prüfschaltung zur Prüfung der Integrität des Regelkreises derart,
daß die Prüfschaltung bei von der Stellvorrichtung gezielt vorgenommenen, kleinen und ggf. kurzzeitigen Änderungen des Sollwertes die Plausibilität der Reaktion des Patienten oder des Systems mit Plausibilitätsdaten vergleicht.

16. System nach einem der vorhergehenden Ansprüche, gekennzeichnet durch
eine Prüfschaltung zur Prüfung der Integrität des Regelkreises derart,
dass die Prüfschaltung dem Messaufnehmer des Patientenmonitors für jeweils kurze Zeiten Testsignale zuführt und den Messwert des Monitors auf Plausibilität prüft.
